# EUROPEAN PATENT APPLICATION

(11) **EP 4 245 351 A1**
(43) Date of publication of application: **20.09.2023**
(21) Application number: 21891640.1
(22) Date of filing: 26.10.2021
(51) Int. Cl.: A61M 39/02, A61M 37/00

(54) **MEDICAL DEVICE, MEDICAL EQUIPMENT COMPONENT, AND MEDICAL EQUIPMENT**

(30) Priority: 10.11.2020 JP 2020187638; 27.01.2021 JP 2021010910
(71) Applicant: FURUKAWA ELECTRIC CO., LTD., Chiyoda-ku Tokyo 100-8322 (JP)
(72) Inventor: SUEMATSU, Katsuki, Tokyo 100-8322 (JP); HIMURA, Atsushi, Tokyo 100-8322 (JP); YAGI, Takeshi, Tokyo 100-8322 (JP); NARA, Kazutaka, Tokyo 100-8322 (JP); ARAI, Tsunenori, Tokyo 100-8322 (JP)
(74) Representative: Winter, Brandl - Partnerschaft mbB
(86) International application number: PCT/JP2021/039444
(87) International publication number: WO 2022/102393

(57) **Abstract**

Provided are a medical device that can easily confirm a predetermined portion in a medical instrument implanted for use in a body, a medical instrument component and a medical instrument. A medical instrument 10 includes a main body part 11, a medicinal solution container 12 installed in the main body part 11, a notification part 17 including a plurality of light emitting portions 17a that emit light by use of power received by a power receiving part 16 to notify that a predetermined state is reached, when a relative positional relation between a power transmission part 22 and the power receiving part 16 reaches the predetermined state by moving the power transmission part 22, and a soft part 13 into which an injection needle is inserted, the plurality of light emitting portions 17a are arranged along an outer edge of the soft part 13, and a first coil 15 is a coreless coil disposed to have an inner diameter larger than an outer diameter of the medicinal solution container 12.

## Description

### TECHNICAL FIELD

The present disclosure relates to a medical device that detects a position of a medical instrument implanted for use in a subject body, a medical instrument component, and a medical instrument.

### BACKGROUND ART

In order to inject a medicinal solution into a body, an implantable medical device including a medical instrument implanted in the body is used. This medical instrument reduces burdens on a patient who frequently needs to have an injection, in injecting the medicinal solution. The medical instrument has, in a main body part, a soft part into which an injection needle is inserted. This soft part is formed from, for example, silicone rubber or the like. Then, the medicinal solution is injected through the soft part into a medicinal solution container of the medical instrument. The medicinal solution is transported to a blood vessel through an infusion tube.

Since the main body part of the medical instrument is implanted in the body, it is difficult to identify a position of the soft part by visual recognition from outside of the body.

Therefore, by applying a technology proposed in Patent Document 1, a medical instrument is conceivable in which a soft part is formed by mixing, with a resin material, a light emitting material that emits near-infrared fluorescence when irradiated with near-infrared excitation light. With such a medical instrument, the position of the soft part may be identified through visual recognition by converting near-infrared fluorescence to visible light.

Further, in an implantable medical device of Patent Document 2, disclosed is a technology of supplying power to a plurality of light sources provided in the medical device by non-contact power supply using a coil, and emitting light from a light source mounted on a medical instrument. According to such a technology, it may be possible to identify a position of the medical device implanted in the body using the light source as a mark.

### PRIOR ART DOCUMENTS

### Patent literatures

Patent Document 1: Japanese Patent No. 5958922
Patent Document 2: US Patent No. 7191011

### DISCLOSURE OF THE INVENTION

### Problems to be Solved by the Invention

However, when a light emitting material is mixed with a resin material to form a soft part, the light emitting material aggregates, an amount of near-infrared fluorescence emitted by the light emitting material is reduced by concentration quenching, and as a result, it might be difficult to identify a position of the soft part.

Herein, description has been made herein as to medical instrument having the soft part, but an implantable medical device that does not have the soft part has a similar problem.

In addition, a plurality of light sources are required to accurately identify a position, inclination, or the like of medical instrument implanted in a body, and a certain or more amount of light is required for light from a light source implanted in the body to reach a body surface. Therefore, a certain or more amount of power supply is required.

To secure the power supply amount, a method of attaching a coil using, in a core, a magnetic material such as ferrite to an individual light source and efficiently supplying power can be considered. However, there is a problem that the magnetic material, such as ferrite, is likely to be an artifact of an MRI image. On the other hand, if the ferrite core is not used, enlargement of the medical instrument itself, for example, increase of a thickness of the medical instrument due to enlargement of a power receiving part including the coil is inevitable.

An object of the present disclosure, which has been made in view of the above, is to provide a medical device that can easily confirm a predetermined portion in a medical instrument implanted for use in a body, a medical instrument component and a medical instrument.

### Means for Solving the Problems

A medical device according to the present invention includes a power transmission part including a second coil that transmits power in a non-contact manner; and a medical instrument implanted for use in a body, and including a power receiving part including a first coil that receives the power transmitted from the power transmission part, wherein the medical instrument includes: a housing; a container installed in the housing; a notification part including a plurality of light emitting portions that emit light by use of the power received by the power receiving part to notify that a predetermined state is reached, when a relative positional relation between the power transmission part and the power receiving part reaches the predetermined state by moving the power transmission part; and a soft part into which an injection needle is inserted, the plurality of light emitting portions are arranged along an outer edge of the soft part, and the first coil is a coreless coil disposed to have an inner diameter larger than an outer diameter of the container.

Also, in the medical device according to the present invention, the plurality of light emitting portions share the power receiving part, and the respective light emitting portions are electrically connected in parallel.

Further, a medical instrument component according to the present invention is a medical instrument component mounted on a medical instrument implanted for use in a body, the medical instrument including a power receiving part including a first coil that receives power transmitted from a power transmission part of an extracorporeal unit in a non-contact manner; and a notification part including a plurality of light emitting portions that emit light by use of the power received by the power receiving part to notify that a predetermined state is reached, when a relative positional relation between the power transmission part and the power receiving part reaches the predetermined state by moving the power transmission part, wherein the first coil is a coreless coil, the power receiving part is installed on one plane of a substrate, and the notification part is installed on another plane of the substrate.

Additionally, in the medical instrument component according to the present invention, the other plane of the substrate, together with the notification part, is covered with a resin material.

Also, a medical instrument according to the present invention is a medical instrument implanted for use in a body, and including a power receiving part including a first coil that receives power transmitted from a power transmission part of an extracorporeal unit in a non-contact manner; a notification part including a plurality of light emitting portions that emit light by use of the power received by the power receiving part to notify that a predetermined state is reached, when a relative positional relation between the power transmission part and the power receiving part reaches the predetermined state by moving the power transmission part; a housing; a container installed in the housing; and a soft part into which an injection needle is inserted, wherein the plurality of light emitting portions are arranged along an outer edge of the soft part, and the first coil is a coreless coil disposed to have an inner diameter larger than an outer diameter of the container.

Further, the medical instrument according to the present invention includes a first exterior part in which the housing includes the container; a second exterior part including an insertion part into which the first exterior part is inserted; and a medical instrument component disposed between the second exterior part and the first exterior part, wherein the power receiving part is installed on one plane, and a notification part is installed on another plane located on a side opposite to the one plane.

Additionally, in the medical instrument according to the present invention, the light emitting portions emit light from the first exterior part toward the second exterior part.

Also, in the medical instrument according to the present invention, the medical instrument component is installed in the first exterior part.

### Effects of the Invention

According to the present disclosure, produced is an effect of being able to easily confirm a predetermined portion in a medical instrument implanted for use in a body.

### BRIEF DESCRIPTION OF THE DRAWINGS

FIG. 1(a) is an exploded perspective view showing a first coil according to a first embodiment of the present invention, and a plurality of light emitting portions installed on a substrate, and
FIG. 1(b) is a perspective view showing an example where the number of light emitting portions installed on the substrate according to the first embodiment of the present invention is different;
FIG. 2(a) is an exploded perspective view of a medical instrument according to the first embodiment of the present invention, and
FIG. 2(b) is an overall perspective view of the medical instrument according to the first embodiment of the present invention;
FIG. 3 is a side cross-sectional view of the medical instrument according to the first embodiment of the present invention;
FIG. 4 is a side cross-sectional view of a medical instrument according to a second embodiment of the present invention;
FIG. 5 is a side cross-sectional view of a medical instrument according to a third embodiment of the present invention;
FIG. 6 is a side cross-sectional view of a medical instrument according to a fourth embodiment of the present invention;
FIG. 7 is a side cross-sectional view of a medical instrument according to a fifth embodiment of the present invention;
FIG. 8(a) is a perspective view of a capacitor and a plurality of light emitting portions installed on a substrate according to a sixth embodiment of the present invention, and
FIG.8(b) is a perspective view explaining that the capacitor and the plurality of light emitting portions installed on the substrate are molded of a transparent resin;
FIG. 9 is a perspective view explaining that a state where a capacitor and a plurality of types of light emitting portions are installed on a substrate according to a seventh embodiment of the present invention;
FIG. 10 is a diagram explaining examples of another extracorporeal unit;
FIG. 11 is a side cross-sectional view of a medical instrument according to an eighth embodiment of the present invention;
FIG. 12 is a side cross-sectional view of a medical instrument according to a ninth embodiment of the present invention;
FIG. 13 is a side cross-sectional view of a medical instrument according to a tenth embodiment of the present invention;
FIG. 14 is a side cross-sectional view of a medical instrument according to an eleventh embodiment of the present invention;
FIG. 15 is a side cross-sectional view of a medical instrument according to a twelfth embodiment of the present invention;
FIG. 16 is a side cross-sectional view of a medical instrument according to a thirteenth embodiment of the present invention;
FIG. 17 relates to a fourteenth embodiment of the present invention, FIG. 17(a) is a side cross-sectional view of a medical instrument, and FIGS. 17(b), 17(c), and 17(d) are views of a surface facing a substrate in a main body part as viewed from below; and
FIG. 18 is a side cross-sectional view of a medical instrument according to a fifteenth embodiment of the present invention.

### PREFERRED MODE FOR CARRYING OUT THE INVENTION

Hereinafter, a mode for carrying out the present invention will be described in detail with drawings. Note that the present invention is not limited by the following embodiments. Also, each figure referred to in the following description only schematically shows a shape, size, and positional relation to such an extent that content of the present invention can be understood. Specifically, the present invention is not limited only to the shape, size, and positional relation illustrated in each figure. Furthermore, in the following description, a medical instrument and a medical instrument implanted for use in a living body of a subject including a human or an animal will be described in detail.

### <First Embodiment>

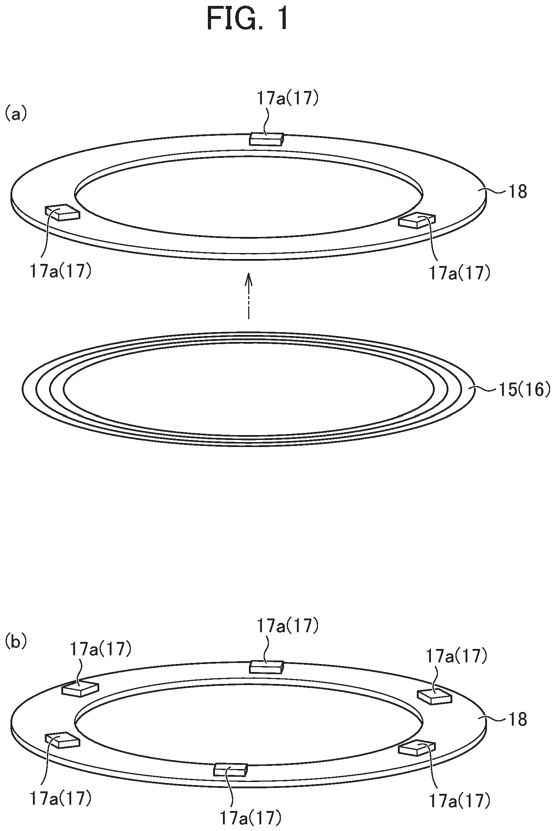

FIGS. 1 to 3 show a first embodiment of the present invention. In the first embodiment, a medical instrument is provided with a notification part, and the notification part notifies that a predetermined state is reached.

### [Configuration of Medical Device]

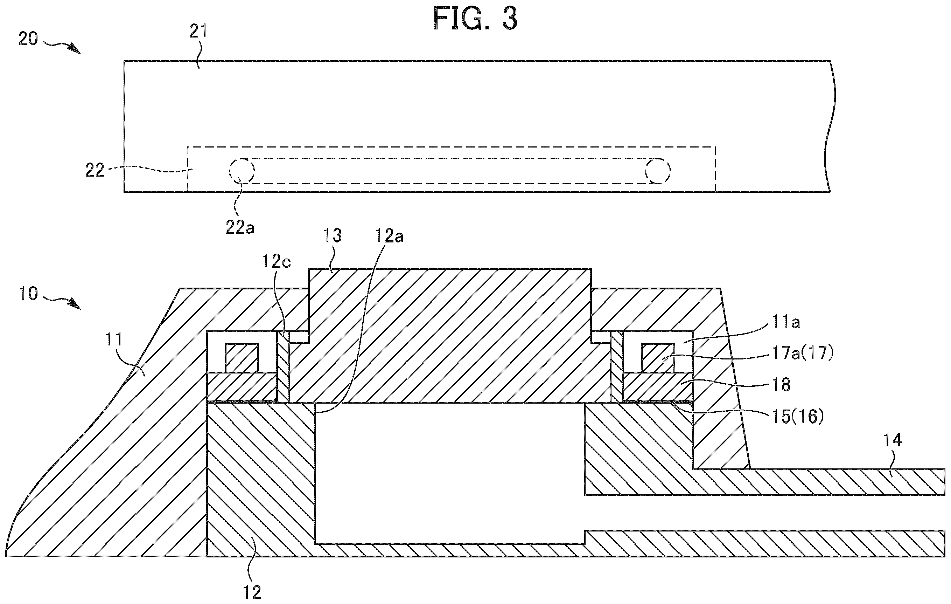

As shown in FIG. 3, a medical device 1 includes a medical instrument 10 implanted for use in a living body of a subject, and an extracorporeal unit 20.

### [Configuration of Medical Instrument]

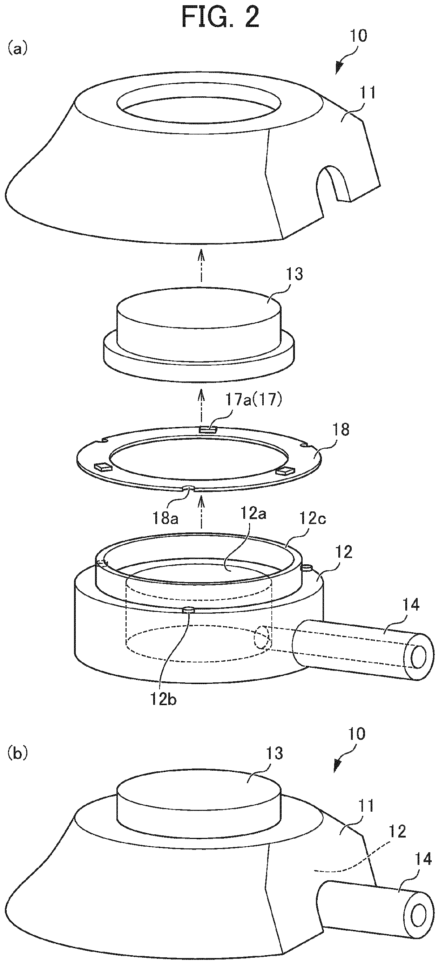

The medical instrument 10 shown in FIGS. 2 and 3 is referred to as, for example, a subcutaneous implantable port (a CV port).

A main body part 11 is a housing made of, for example, an epoxy resin or the like. The main body part 11 includes a medicinal solution container 12, a soft part 13, an infusion tube 14, a power receiving part 16 including a first coil 15, and a notification part 17 that notifies that a relative positional relation between the first coil 15 and an after-mentioned power transmission part reaches the predetermined state by use of power received by the power receiving part 16. Also, in the main body part 11, an insertion portion 11a, into which the power receiving part 16 and the notification part 17 are inserted, may be formed. A sealed space is formed in the insertion portion 11a by welding the main body part 11, the medicinal solution container 12 and the soft part 13 to one another.

The medicinal solution container 12 is a chamber that temporarily stores a medicinal solution. The medicinal solution container 12 has an opening 12a having a circular shape outside the body, and the infusion tube 14 connecting an unshown catheter. Further, on the upper surface of the medicinal solution container 12, a plurality of protrusions 12b engaged in notches provided in an after-mentioned substrate are provided at intervals in a circumferential direction. Furthermore, on an upper surface of the medicinal solution container 12, a partition wall 12c separating between the soft part 13 and the after-mentioned substrate is provided.

The soft part 13 is a so-called septum. Then, the soft part 13 closes the opening 12a in the medicinal solution container 12. The soft part 13 is, for example, a soft lid body (silicone diaphragm) made of silicone rubber and is provided in an exposed manner also from the main body part 11. Further, the soft part 13 is cylindrical. The soft part 13 is a part into which an injection needle for medicinal solution injection (infusion) can be inserted from a body surface of the subject after the main body part 11 is implanted in the living body.

The infusion tube 14 has one end communicating with the medicinal solution container 12, and the other end connected to the unshown catheter, and the catheter is inserted into an unshown blood vessel or the like. The infusion tube 14 transports the medicinal solution temporarily stored in the medicinal solution container 12.

The first coil 15 is formed by winding an electric wire in an annular shape a plurality of times without having a core of ferrite (magnetic material) or the like. The first coil 15 has an inner diameter larger than an outer diameter of the medicinal solution container 12.

The power receiving part 16 includes the first coil 15 and a power receiving circuit. The first coil 15 receives the power transmitted from a power transmission part 22. In the power receiving circuit, a capacitor 17c may be used. The power generated in the first coil 15 (induced electromotive force) is received, and the received power is supplied to an after-mentioned light emitting portion of the notification part 17. The power receiving part 16 and the notification part 17 may be provided on the same substrate, and as shown in FIG. 1(a), the power receiving part 16 may be installed by being affixed to one plane of a substrate 18 formed in a ring shape, or some components including the capacitor 17c may be installed on a plane on an opposite side. Alternatively, a circuit pattern may be integrally formed in a coil shape by etching or the like as in a printed circuit board (PCB), and in this case, disconnection risk at the time of component handling can be reduced, and furthermore, manufacturing cost at the time of mass production can be reduced. Further, on an outer periphery of the substrate 18, a plurality of notches 18a, in which a plurality of protrusions 12b of the medicinal solution container 12 engage, are provided at intervals in the circumferential direction.

The notification part 17 includes a plurality of light emitting portions 17a each including an LED chip. The plurality of light emitting portions 17a share the power receiving part 16, and the respective light emitting portions 17a are electrically connected in parallel.

For example, as shown in FIG. 1(a), the plurality of light emitting portions 17a are arranged at predetermined intervals along an outer edge of the soft part 13 by installing the light emitting portions 17a on the other plane of the substrate 18. Specifically, the light emitting portions 17a are arranged in three portions in an annular shape along the outer edge of the soft part 13 every 120 degrees around a center of the first coil 15. The light emitting portions 17a emit light depending on the power received by the power receiving part 16. In the light emitting portions 17a, LEDs having a high directivity are preferably used. More specifically, in the light emitting portions 17a, red LEDs that emit light in a red wavelength band are preferably used for transmission through the living body of the subject or the like. Note that the number and arrangement of the light emitting portions 17a may be appropriately changed and, for example, as shown in FIG. 1(b), the light emitting portions 17a may be arranged in six portions along the outer edge of the soft part 13 in the annular shape at equal intervals around the center of the first coil 15.

Here, when an exterior part including the medicinal solution container 12 is a first exterior part and the main body part 11 including the insertion portion 11a into which the first exterior part is inserted is a second exterior part, the medical instrument includes a medical instrument component disposed between the first exterior part and the second exterior part, wherein the power receiving part 16 is installed on one plane of the substrate 18, and the notification part is installed on the other plane of the substrate.

### [Configuration of Extracorporeal Unit]

As shown in FIG. 3, the extracorporeal unit 20 includes a housing 21 and the power transmission part 22. The power transmission part 22 includes a second coil 22a. The housing 21 and the power transmission part 22 are connected in a wired or wireless manner. In the wired manner, a form is considered where power is supplied from the housing 21 to the power transmission part 22 by the connection with a cable having a predetermined length. Further, in the wireless manner, for example, a form is considered where power is supplied from the housing 21 to the power transmission part 22 by use of non-contact power supply.

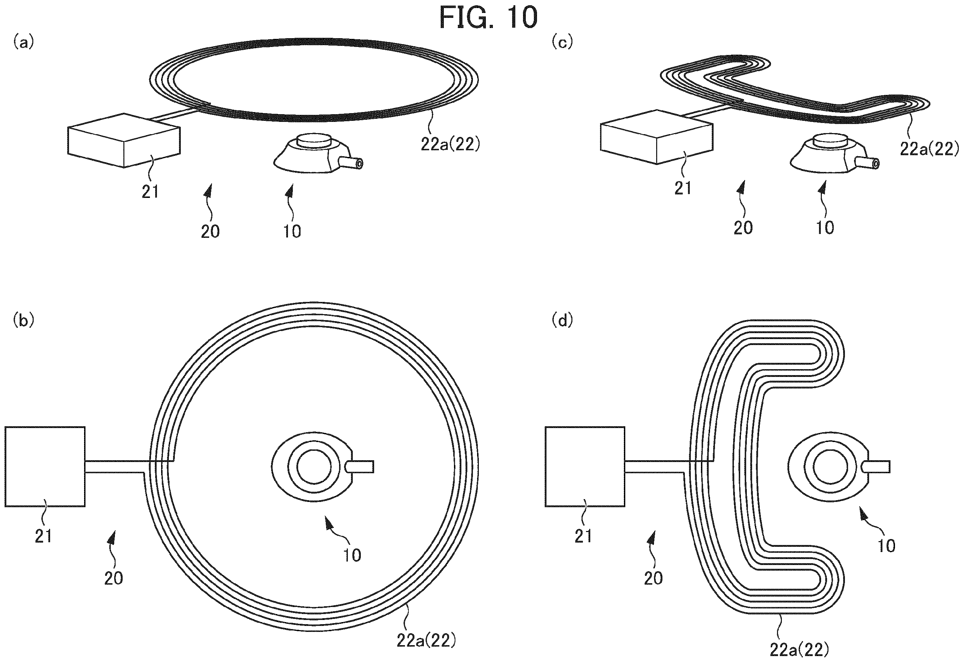

As shown in FIGS. 10(a) and (b), the second coil 22a has an annular shape, generates a magnetic flux depending on the power inputted from an unshown power source, and transmits the power to the first coil 15. Also, power is supplied through the second coil 22a to such an extent that the plurality of light emitting portions 17a can emit light, even if a distance between the second coil 22a and the first coil 15 is a predetermined distance, for example, a distance of about several tens of cm. Further, as shown in FIGS. 10(c) and (d), a dent may be formed with the second coil. At this time, the dent may be larger than the soft part 13 and may only have a space where puncture is possible. This makes it easier to remove the second coil after power supply.

The medical device 1 configured as above supplies power from the extracorporeal unit 20 and allows the plurality of light emitting portions 17a in the medical instrument 10 to emit light, thereby notifying a place where the medical instrument 10 is implanted. Consequently, a user can intuitively grasp a position and center of the soft part 13 in the medical instrument 10 from outside the subject.

According to the first embodiment, the plurality of light emitting portions 17a in the medical instrument 10 emit light to notify the position of the soft part 13, and hence the position of the soft part 13 in the medical instrument 10 can be intuitively grasped from outside the subject.

Thus, the medical device 1 of the present embodiment includes the power transmission part 22 including the second coil 22a that transmits power in a non-contact manner; and the medical instrument 10 implanted for use in a body, and including the power receiving part 16 including the first coil 15 that receives the power transmitted from the power transmission part 22, the medical instrument 10 including the main body part 11; the medicinal solution container 12 installed in the main body part 11; the notification part 17 including the plurality of light emitting portions 17a that emit light by use of the power received by the power receiving part 16 to notify that the predetermined state is reached, when a relative positional relation between the power transmission part 22 and the power receiving part 16 reaches the predetermined state by moving the power transmission part 22; and the soft part 13 into which the injection needle is inserted, wherein the plurality of light emitting portions 17a are arranged along the outer edge of the soft part 13, and the first coil 15 is a coreless coil disposed to have the inner diameter larger than the outer diameter of the medicinal solution container 12.

Also, it is preferable that the plurality of light emitting portions 17a share the power receiving part 16, and the respective light emitting portions 17a are electrically connected in parallel.

Further, a medical instrument component according to the present invention is a medical instrument component mounted on the medical instrument 10 implanted for use in the body, the medical instrument including the power receiving part 16 including the first coil 15 that receives power transmitted from the power transmission part 22 of the extracorporeal unit 20 in a non-contact manner, and the notification part 17 including the plurality of light emitting portions 17a that emit light by use of the power received by the power receiving part 16 to notify that the predetermined state is reached, when the relative positional relation between the power transmission part 22 and the power receiving part 16 reaches the predetermined state by moving the power transmission part 22, and it is preferable that the first coil is the coreless coil, the power receiving part 16 and the notification part 17 may be installed on the same substrate, the power receiving part 16 is installed on one plane of the substrate 18, and the notification part 17 is installed on the other plane of the substrate 18.

Also, the medical instrument 10 according to the present invention is the medical instrument 10 implanted for use in the body, and including the power receiving part 16 including the first coil 15 that receives power transmitted from the power transmission part 22 of the extracorporeal unit 20 in a non-contact manner; the notification part 17 including the plurality of light emitting portions 17a that emit light by use of the power received by the power receiving part 16 to notify that the predetermined state is reached, when the relative positional relation between the power transmission part 22 and the power receiving part 16 reaches the predetermined state by moving the power transmission part 22; the main body part 11; the medicinal solution container 12 installed in the main body part 11; and the soft part 13 into which the injection needle is inserted, the plurality of light emitting portions 17a are arranged along the outer edge of the soft part 13, and the first coil 15 is the coreless coil disposed to have the inner diameter larger than the outer diameter of the medicinal solution container 12. Further, the first coil preferably has an inner diameter of 15 mm or more and an outer diameter of 21 mm or less, depending on a size of the medical instrument. With such diameters, it is possible to secure a predetermined amount of power supply and make the medical instrument thinner.

Additionally, it is preferable that the medical instrument 10 according to the present invention includes the first exterior part including the medicinal solution container 12; the second exterior part including the insertion portion 11a into which the first exterior part is inserted; and the medical instrument component disposed between the second exterior part and the first exterior part, wherein the power receiving part 16 is installed on one plane of the substrate 18, and the notification part 17 is installed on the other plane of the substrate 18. Further, according to the medical instrument 10 of the present invention, an exterior of the medical instrument may include two exterior parts that can engage each other. Furthermore, the insertion portion 11a in which the notification part 17 and the first coil 15 can be installed may be formed between two engageable exterior bodies, and it is more preferable that the insertion portion 11a is provided in one exterior part where the soft part 13 is exposed, and a fixing part for the soft part 13, the notification part 17 and the first coil 15 is provided on the other exterior part. According to such a configuration, fixing of the soft part 13, the notification part 17 and the first coil 15, and fitting and welding of the exterior parts can be performed at the same time. More specifically, it is preferable to include a first exterior part including the medicinal solution container 12; a second exterior part where the soft part 13 is exposed; a medical instrument component disposed between the second exterior part and the first exterior part, wherein the power receiving part 16 including the first coil 15 is installed on one plane of the substrate 18 and the notification part 17 is installed on the other plane of the substrate 18 in the first exterior part; and a support part in which at least a part of each of the soft part 13 and the power receiving part 16 is supported with the first exterior part. Further, a fitting part may be provided in which a substrate of a medical instrument component with the power receiving part 16 including the first coil 15 being installed on one plane of the substrate 18 and the notification part 17 being installed on the other plane of the substrate 18 is fitted with the support part of the first exterior part. More specifically, protrusions installed on the support part and grooves formed in the substrate are provided.

Also, according to the medical instrument 10 of the present invention, it is preferable that the light emitting portions 17a emit light from the first exterior part including the medicinal solution container 12 toward the second exterior part in which the soft part 13 is installed.

### <Second Embodiment>

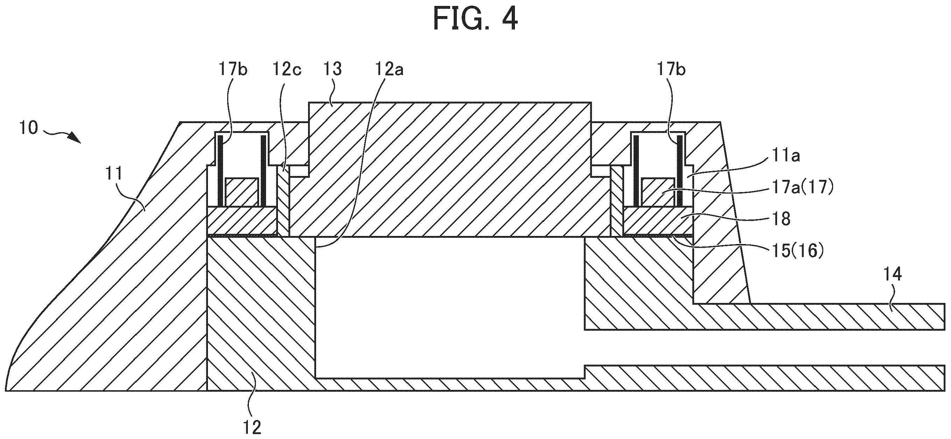

FIG. 4 shows a second embodiment of the present invention. Constituent parts similar to those of the first embodiment are denoted with the same reference numerals and are not described in detail.

A medical instrument 10 of the second embodiment includes a light guide plate 17b on an outer peripheral side of a light emitting portion 17a. The light guide plate 17b is installed vertically from a plane of a substrate 18 on which the light emitting portion 17a is provided. The light guide plate 17b may be replaced with a light shielding plate. The light shielding plate can further reduce diffusion of light and improve visibility under a constant environment.

Consequently, light emitted from the light emitting portion 17a travels toward a surface where a soft part 13 of a main body part 11 exists without being diffused, so that user's visibility improves.

At this time, a convex portion or a concave portion in which the light guide plate 17b engages may be formed on a surface of the main body part 11 that faces the light emitting portion 17a. In this case, a medicinal solution container 12 is positioned in a rotating direction, and it is possible to suppress occurrence of misalignment, in the rotating direction, of the medicinal solution container 12 with respect to the main body part 11.

### <Third Embodiment>

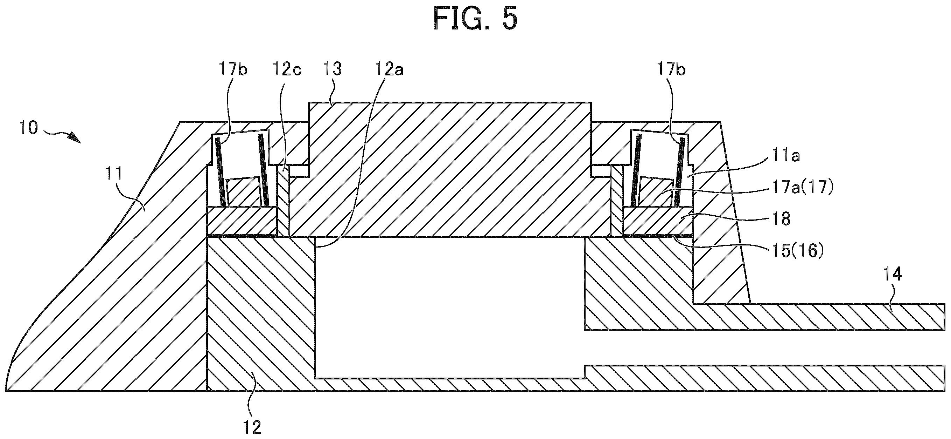

FIG. 5 shows a third embodiment of the present invention. Constituent parts similar to those of the first and second embodiments are denoted with the same reference numerals and are not described in detail.

A medical instrument 10 of the third embodiment is installed so that a light guide plate 17b provided on an outer periphery of a light emitting portion 17a and a light guide plate 17b provided on an outer periphery of another light emitting portion 17a are oriented in different directions.

Consequently, for light emitted from the light emitting portions 17a, interference with light emitted from a plurality of light emitting portions 17a around a soft part 13 can be suppressed, and hence the user's visibility improves.

### <Fourth Embodiment>

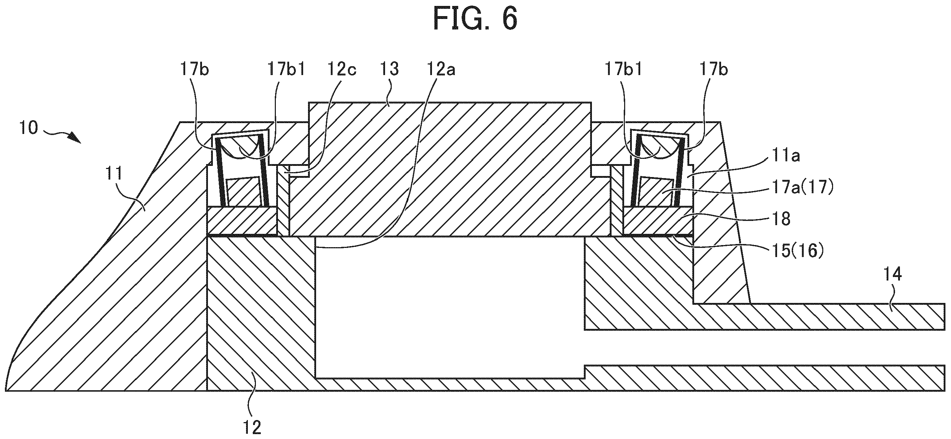

FIG. 6 shows a fourth embodiment of the present invention. Constituent parts similar to those of the first to third embodiments are denoted with the same reference numerals and are not described in detail.

A medical instrument 10 of the fourth embodiment includes a condensing portion 17b1 having a curved surface facing a light emitting portion 17a, in a light guide plate 17b provided on an outer periphery of the light emitting portion 17a.

Consequently, light emitted from the light emitting portion 17a travels toward a surface where a soft part 13 of a main body part 11 exists without being diffused, and user's visibility improves. A direction of the light guide plate 17b can be determined in the same manner as in the second or third embodiment.

<Fifth Embodiment>

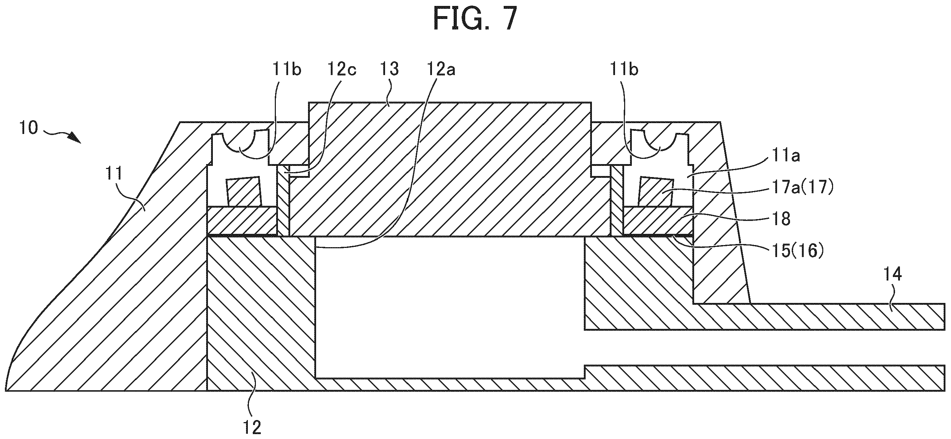

FIG. 7 shows a fifth embodiment of the present invention. Constituent parts similar to those of the first to fourth embodiments are denoted with the same reference numerals and are not described in detail.

In a medical instrument 10 of the fifth embodiment, in a main body part 11, a condensing portion 11b having a curved surface facing a light emitting portion 17a is formed.

Consequently, light emitted from the light emitting portion 17a is guided toward a surface where a soft part 13 of the main body part 11 exists without being diffused, and user's visibility improves.

A light guide plate 17b may be installed on an outer periphery of the light emitting portion 17a as in the second or third embodiment.

### <Sixth Embodiment>

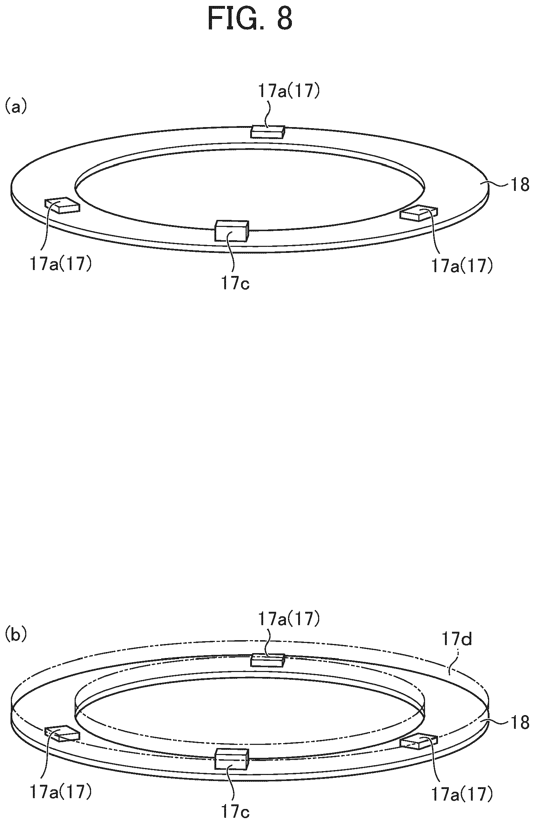

FIG. 8 shows a sixth embodiment of the present invention. Constituent parts similar to those of the first to fifth embodiments are denoted with the same reference numerals and are not described in detail.

In a medical instrument 10 of a sixth embodiment, a power receiving part 16 is provided on one plane of a substrate 18, and a plurality of light emitting portions 17a serving as a notification part 17 are provided on the other plane of the substrate 18. At this time, a capacitor 17c may be installed on the plane on the same side as the plurality of light emitting portions 17a. Also, the plurality of light emitting portions 17a, capacitor 17c and a wiring installed on the other plane of the substrate 18 are covered with a transparent resin material 17d.

Consequently, the light emitting portions 17a, the capacitor 17c and wiring installed on a flat surface of the substrate 18 are covered with the resin material, and hence it is possible to prevent damages on components during assembling of the medical instrument 10.

### <Seventh Embodiment>

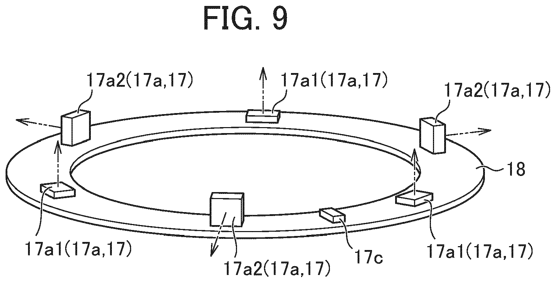

FIG. 9 shows a seventh embodiment of the present invention. Constituent parts similar to those of the first to sixth embodiments are denoted with the same reference numerals and are not described in detail.

In a medical instrument 10 of the seventh embodiment, a power receiving part 16 is provided on one plane of a substrate 18, and a capacitor 17c is provided, together with a plurality of light emitting portions 17a serving as a notification part 17, on the other plane of the substrate 18. Further, the plurality of light emitting portions 17a installed on the other plane of the substrate 18 have a plurality of first light emitting portions 17a1 that emit light in a direction perpendicular to the plane of the substrate 18, and a plurality of second light emitting portions 17a2 that emit light in an outer peripheral direction of the substrate 18.

Consequently, power can be supplied to the plurality of first light emitting portions 17a1 and the plurality of second light emitting portions 17a2 for different applications with one first coil 15.

### <Eighth Embodiment>

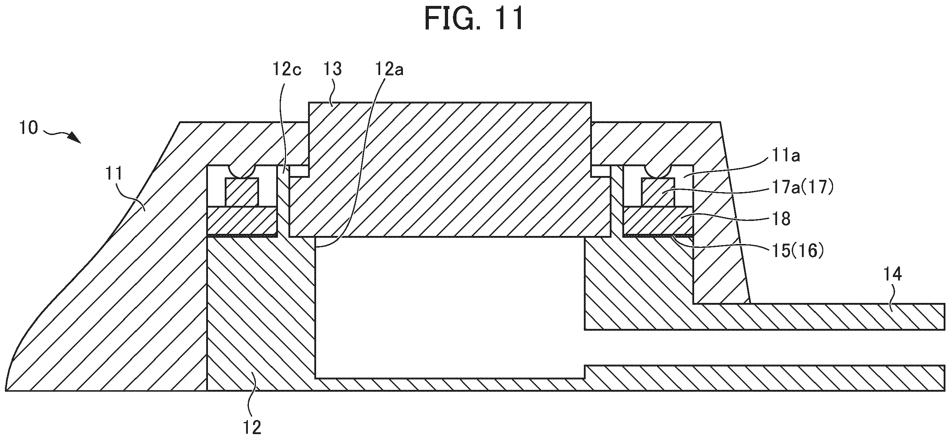

FIG. 11 shows an eighth embodiment of the present invention. Constituent parts similar to those of the first to seventh embodiments are denoted with the same reference numerals and are not described in detail.

In a medical instrument 10 of the eighth embodiment, as shown in FIG. 11, a medicinal solution container 12 and a partition wall 12c are integrally formed.

Consequently, any gap is not formed between the medicinal solution container 12 and the partition wall 12c, and pressure resistance of the medical instrument 10 can be improved. Further, the medicinal solution container 12 and the partition wall 12c form one component, and hence the assembling man-hours can be reduced.

### <Ninth Embodiment>

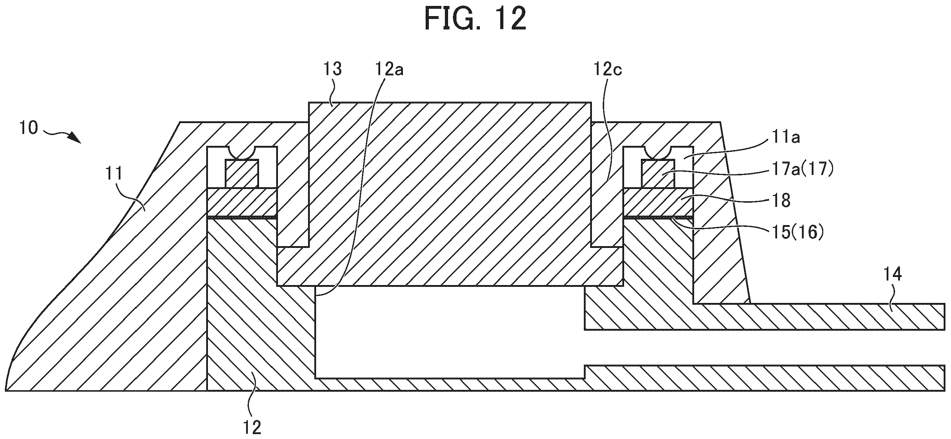

FIG. 12 shows a ninth embodiment of the present invention. Constituent parts similar to those of the first to eighth embodiments are denoted with the same reference numerals and are not described in detail.

In a medical instrument 10 of the ninth embodiment, as shown in FIG. 12, a main body part 11 and a partition wall 12c are integrally formed. Further, a lower end portion of the partition wall 12c abuts on a soft part 13 over a circumferential direction.

Consequently, a contact area between the main body part 11 and the soft part 13 can be increased, and pressure resistance of the medical instrument 10 can be improved. Further, the main body part 11 and the partition wall 12c form one component, and hence assembly man-hours can be reduced.

### <Tenth Embodiment>

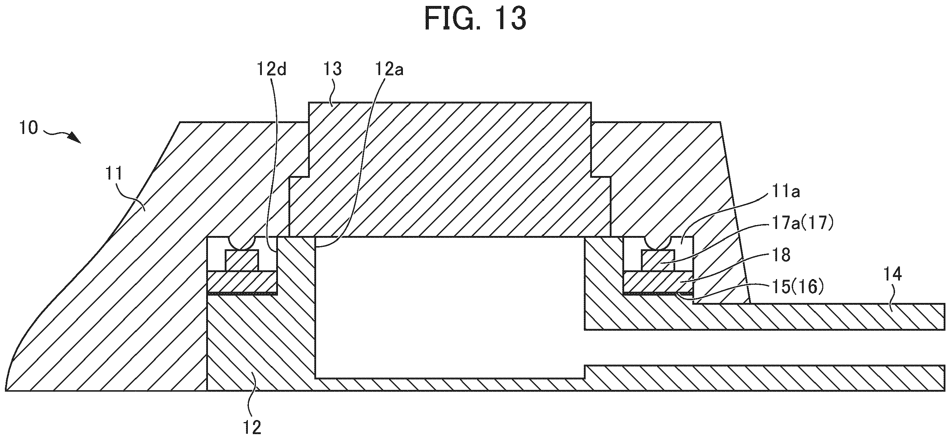

FIG. 13 shows a tenth embodiment of the present invention. Constituent parts similar to those of the first to ninth embodiments are denoted with the same reference numerals and are not described in detail.

A medical instrument 10 of the tenth embodiment does not have a partition wall 12c. A notch portion 12d is formed over a circumferential direction on an outer peripheral side of an upper side of a medicinal solution container 12 of the tenth embodiment. In the notch portion 12d, a first coil 15, a plurality of light emitting portions 17a and a substrate 18 are installed. The first coil 15, the plurality of light emitting portions 17a and the substrate 18 installed in the notch portion 12d are housed in a space surrounded with a main body part 11 and the medicinal solution container 12 by assembling the main body part 11 and the medicinal solution container 12 together.

Thus, according to the medical instrument of the present embodiment, in a case where an exterior part including the medicinal solution container 12 is a first exterior part and the main body part 11 including an insertion portion 11a into which the first exterior part is inserted is a second exterior part, the first coil 15, the plurality of light emitting portions 17a and the substrate 18 are installed in the first exterior part.

Consequently, without requiring the partition wall 12c, it is possible to suppress permeation of a medicinal solution into a side of the first coil 15, the plurality of light emitting portions 17a and the substrate 18 with a simple configuration, and manufacturing cost can be reduced.

Note that the tenth embodiment illustrates that the first coil 15, the plurality of light emitting portions 17a, and the substrate 18 are installed in the notch portion 12d formed over the circumferential direction on the outer peripheral side on the upper side of the medicinal solution container 10 but is not limited to this configuration. The first coil 15, the plurality of light emitting portions 17a and the substrate 18 may only be installed in the medicinal solution container 10, and may be installed, for example, in a groove formed over the circumferential direction on the upper side of the medicinal solution container 10.

### <Eleventh Embodiment>

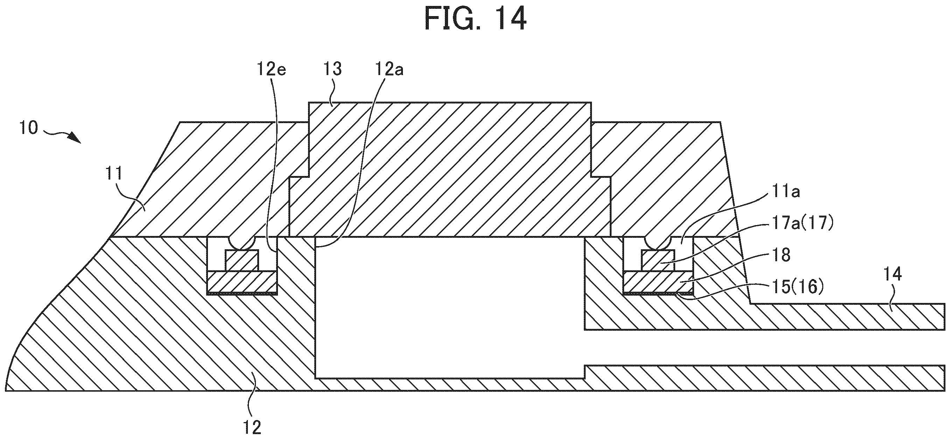

FIG. 14 shows an eleventh embodiment of the present invention. Constituent parts similar to those of the first to tenth embodiments are denoted with the same reference numerals and are not described in detail.

A medical instrument 10 of the eleventh embodiment does not include a partition wall 12c. In an upper surface of a medicinal solution container 12 of the eleventh embodiment, a groove 12e extending in a circumferential shape is formed. In the groove 12e, a first coil 15, a plurality of light emitting portions 17a and a substrate 18 are installed. The first coil 15, the plurality of light emitting portions 17a and the substrate 18 installed in the groove 12e are housed in a space surrounded with a main body part 11 and the medicinal solution container 12 by assembling the main body part 11 and the medicinal solution container 12 together.

Consequently, without requiring the partition wall 12c, it is possible to suppress permeation of a medicinal solution into a side of the first coil 15, the plurality of light emitting portions 17a and the substrate 18 with a simple configuration, and manufacturing cost can be reduced.

### <Twelfth Embodiment>

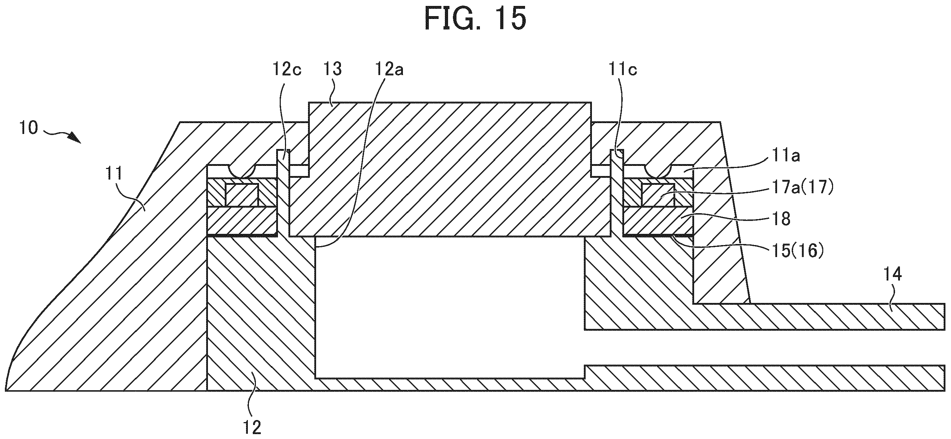

FIG. 15 shows a twelfth embodiment of the present invention. Constituent parts similar to those of the first to eleventh embodiments are denoted with the same reference numerals and are not described in detail.

In a medical instrument 10 of the twelfth embodiment, as shown in FIG. 15, a medicinal solution container 12 and a partition wall 12c are integrally formed. Further, in a lower surface of a main body part 11, a fitting groove 11c, into which an upper end side of the partition wall 12c fits, is formed in a circumferential shape. The upper end side of the partition wall 12c is fitted in the fitting groove 11c over a circumferential direction by assembling the main body part 11 and the medicinal solution container 12 together.

Consequently, a space between the medicinal solution container 12 and the partition wall 12c, and a space between the main body part 11 and the partition wall 12c are closed, respectively, so that pressure resistance of the medical instrument 10 can be further improved. Further, the medicinal solution container 12 and the partition wall 12c form one component, and hence assembly man-hours can be reduced.

### <Thirteenth Embodiment>

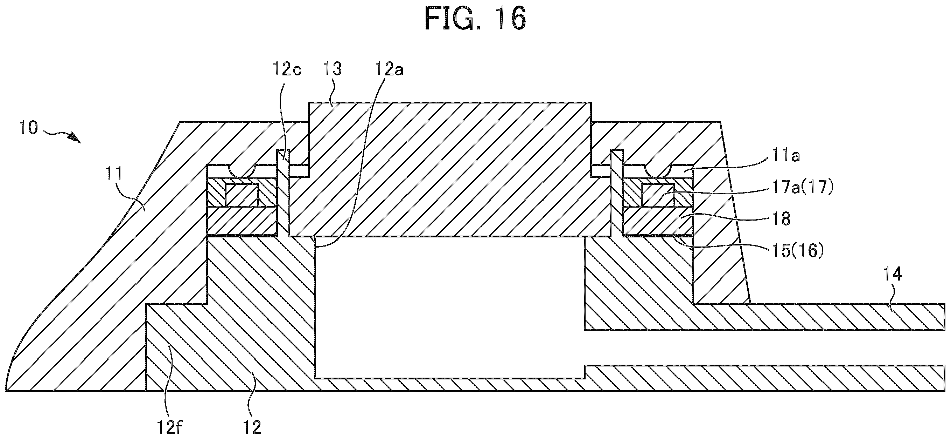

FIG. 16 shows a thirteenth embodiment of the present invention. Constituent parts similar to those of the first to twelfth embodiments are denoted with the same reference numerals and are not described in detail.

As shown in FIG. 16, a medical instrument 10 of the thirteenth embodiment includes a partition wall 12c, and an overhang portion 12f overhanging outward radially over a circumferential direction is formed on a lower side of a side surface of a medicinal solution container 12. Further, a step is formed inside a main body part 11 so that an upper side and the overhang portion 12f of the medicinal solution container 12 are fitted, respectively.

Consequently, a contact area between the main body part 11 and the medicinal solution container 12 can be increased, and hence pressure resistance of the medical instrument 10 can be further improved.

### <Fourteenth Embodiment>

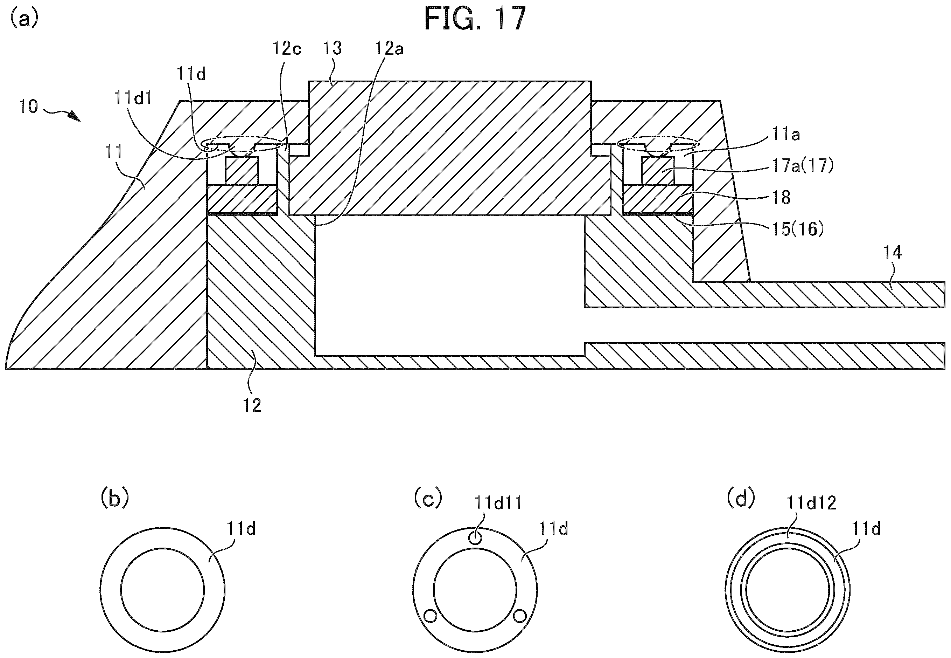

FIG. 17 shows a fourteenth embodiment of the present invention. Constituent parts similar to those of the first to thirteenth embodiments are denoted with the same reference numerals and are not described in detail.

In a medical instrument 10 of the fourteenth embodiment, as shown in FIG. 17(a), a condensing portion 11d1 for condensing light emitted from a light emitting portion 17a provided on a substrate 18 is formed to protrude downward from a surface 11d facing the substrate 18 in a main body part 11.

Consequently, the light emitted from the light emitting portion 17a is condensed by the condensing portion 11d1, and hence it is possible to improve visibility from outside of a subject without requiring adjustment of an amount of light to be emitted from the light emitting portion 17a.

Here, FIGS. 17(b), 17(c) and 17(d) are views of the surface 11d facing the substrate 18 in the main body part 11 as viewed from below, respectively, and relates to the condensing portion 11d1.

FIG. 17(b) shows that the surface 11d is formed in a flat surface shape that does not have the condensing portion 11d1. In this case, there is no effect of condensing light emitted from the light emitting portion 17a, but a structure is simple advantageously in terms of manufacturing cost.

FIG. 17(c) shows a surface 11d including a condensing portion 11d11 formed only at a position that faces the light emitting portion 17a. In this case, a condensing effect of light emitted from the light emitting portion 17a is high, and hence the visibility of the subject from outside can be further improved.

FIG. 17(d) shows a surface 11d including a condensing portion 11d12 formed over a circumferential direction at a position that faces the light emitting portion 17a in a radial direction of the main body part 11. In this case, there is no need for alignment in the circumferential direction between the light emitting portion 17a and the condensing portion 11d12, so that assembling is easy and manufacturing cost can be reduced.

### <Fifteenth Embodiment>

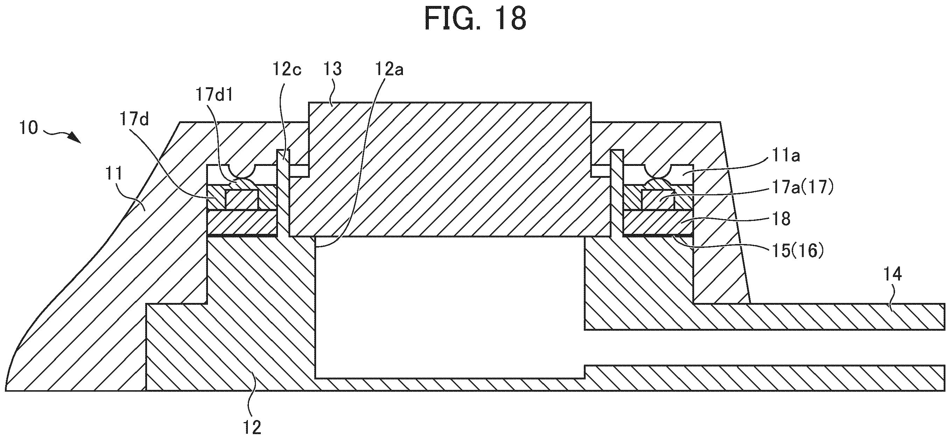

FIG. 18 shows a fifteenth embodiment of the present invention. Constituent parts similar to those of the first to fourteenth embodiments are denoted with the same reference numerals and are not described in detail.

In a medical instrument 10 of the fifteenth embodiment, an upper surface of a substrate 18, together with a light emitting portion 17a, is covered with a transparent resin material 17d for the purpose of protecting a wiring and components provided on the substrate 18. In the resin material 17d, a condensing portion 17d1 for condensing light emitted from the light emitting portion 17a is formed to protrude from a portion located above the light emitting portion 17a.

Consequently, according to a medical instrument component of the present embodiment, the upper surface of the substrate 18, together with the light emitting portion 17a, is covered with the resin material 17d.

Consequently, when the upper surface of the substrate 18 is covered with the resin material 17d, the condensing portion 17d1 can be formed at the same time, so that the condensing portion does not need to be separately formed and manufacturing cost can be reduced.

As above, some of embodiments of the present application have been described in detail based on drawings, but these are illustrations, and it is possible to carry out the present invention in other forms that have undergone various deformations and improvements based on knowledge of those skilled in the art, including aspects described in paragraphs of disclosure of the present invention.

### EXPLANATION OF REFERENCE NUMERALS

1 medical device
10 medical instrument
11 main body part
11a insertion portion
11b condensing portion
12 medicinal solution container
13 soft part
15 first coil
16 power receiving part
17 notification part
17a light emitting portion
18 substrate
20 extracorporeal unit
22 power transmission part
22a second coil

## Claims

1. A medical device comprising:
a power transmission part including a second coil that transmits power in a non-contact manner; and
a medical instrument implanted for use in a body, and including a power receiving part including a first coil that receives the power transmitted from the power transmission part,
wherein the medical instrument includes:
a housing;
a container installed in the housing;
a notification part including a plurality of light emitting portions that emit light by use of the power received by the power receiving part to notify that a predetermined state is reached, when a relative positional relation between the power transmission part and the power receiving part reaches the predetermined state by moving the power transmission part; and
a soft part into which an injection needle is inserted,
the plurality of light emitting portions are arranged along an outer edge of the soft part, and
the first coil is a coreless coil disposed to have an inner diameter larger than an outer diameter of the container.

2. The medical device according to claim 1, wherein
the plurality of light emitting portions share the power receiving part, and the respective light emitting portions are electrically connected in parallel.

3. A medical instrument component mounted on a medical instrument implanted for use in a body,
the medical instrument including: a power receiving part including a first coil that receives power transmitted from a power transmission part of an extracorporeal unit in a non-contact manner; and a notification part including a plurality of light emitting portions that emit light by use of the power received by the power receiving part to notify that a predetermined state is reached, when a relative positional relation between the power transmission part and the power receiving part reaches the predetermined state by moving the power transmission part,
wherein the first coil is a coreless coil,
the power receiving part is installed on one plane of a substrate, and
the notification part is installed on another plane of the substrate.

4. The medical instrument component according to claim 3, wherein
the other plane of the substrate, together with the notification part, is covered with a resin material.

5. A medical instrument implanted for use in a body, and comprising:
a power receiving part including a first coil that receives power transmitted from a power transmission part of an extracorporeal unit in a non-contact manner;
a notification part including a plurality of light emitting portions that emit light by use of the power received by the power receiving part to notify that a predetermined state is reached, when a relative positional relation between the power transmission part and the power receiving part reaches the predetermined state by moving the power transmission part;
a housing;
a container installed in the housing; and
a soft part into which an injection needle is inserted,
wherein the plurality of light emitting portions are arranged along an outer edge of the soft part, and
the first coil is a coreless coil disposed to have an inner diameter larger than an outer diameter of the container.

6. The medical instrument according to claim 5, comprising:
a first exterior part comprising the container;
a second exterior part comprising an insertion part into which the first exterior part is inserted; and
a medical instrument component disposed between the second exterior part and the first exterior part, wherein the power receiving part is installed on one plane of a substrate, and a notification part is installed on another plane of the substrate.

7. The medical instrument according to claim 6, wherein
the light emitting portions emit light from the first exterior part toward the second exterior part.

8. The medical instrument according to claim 6 or 7, wherein
the medical instrument component is installed in the first exterior part.
